# EUROPEAN PATENT SPECIFICATION

(11) **EP 2 737 850 B1**
(45) Date of publication and mention of the grant of the patent: **02.08.2017**
(21) Application number: 13195292.1
(22) Date of filing: 02.12.2013
(51) Int. Cl.: A61B 5/00, A61B 5/055, G01R 33/28, G01R 33/38

(54) **Gantry for mobilizing an MRI device towards static patients**
Gerüst zur Mobilisierung einer MRT-Vorrichtung in Richtung statischer Patienten
Portique pour mobiliser un dispositif IRM en direction de patients statiques

(30) Priority: 02.12.2012 US 201261732353 P
(43) Date of publication of application: 04.06.2014
(73) Proprietor: Aspect Imaging Ltd., 60850 Shoham (IL)
(72) Inventor: Rabinovitz, Itzchak, Ness Tsiyona, 20125 (IL); Toledo, Eran, Tel Aviv, 69027 (IL); Sutton, Thomas Rangi, Milano 20125 (IT); Rapoport, Uri, 73115 (IL)
(74) Representative: Pearl Cohen Zedek Latzer Baratz UK LLP

(56) References cited:
- DE-A1-102008 009 673
- DE-A1-102008 009 674
- US-A1- 2002 123 681
- US-B1- 6 278 274

## Description

### FIELD OF THE INVENTION

The present invention relates to the field of mobilizing an MRI device (MRD). More specifically, it means and methods for approaching a maneuverable MRD towards a static patient to be imagined.

### BACKGROUND OF THE INVENTION

MRI devices are of special importance in the world of medical imaging. Unlike other imaging methods, they present no radiation to the object of which they image. However, together with the benefit of no-radiation, comes the price of very large and complicated machine. In case of patients that are not able to move (e.g. premature baby in an incubator), the option of using an MRI device for scanning is practically impossible.

US patent application US2007/0238950 discloses a maneuverable medical device. However, the constructions of these modules depend on the location of a static patient pallet, and are oriented according to this static pallet location. In addition, the construction and medical devcie requires the entire room to be designated for the imaging.

US 8,555,578 disclose a movable door element in a door of a multimodality medical suite, the door is part of a wall positioned between a first room and a second room of the suite, with the suite including a flexible raceway configured to extend within the length of the suite between the rooms and selectively extend through a portion of the door.

US patent 8,118,488 discloses a medical imaging system, comprising: a base having a top surface, a first end and a second end; a pedestal connected to the base and positioned adjacent to the first end of the base, the pedestal extending in a vertical direction above the top surface of the base and configured to accept a plurality of interchangeable procedure-specific tabletop supports, and the pedestal is rotatable about an axis extending generally normal to the top surface of the base; and a gantry ring having a bore and an image collection apparatus, the gantry ring coupled to the base and positioned above the top surface of base, the gantry ring configured to translate along a length of the base between a first position adjacent to the pedestal to a second position adjacent to the second end of the base, the gantry ring further configured to rotate at least about ninety degrees with respect to an axis extending generally normal to the top surface. This device requires that the patient pallet will be located on the system. In addition, the system is not and cannot be an integral part of a patient's room. The system needs to be delivered to patient's room, and therefore will occupy needed space. Therefore, the system does not support the needs of easily imaging a non-movable patient.

Patent documents DE 10 2008 009674 A1, US 2002/0123681 A1, DE 10 2008 009 673 and US 6,278,274 B1 each disclose embodiments of displacement systems for magnetic resonance probes permanently connected thereto.

There is therefore a long unmet need for a system which is integrated to a patient room, and allows in situ and real time imaging of patients without moving them to remote MRI rooms.

### SUMMARY OF THE INVENTION

Embodiments of the present invention provide a system comprising:
a portable open-bore wrist-type magnetic resonance device (MRD) with no fringing of its magnetic field in which the open bore comprises an aperture,
a gantry for maneuvering said MRD from at least one first location towards at least one second location with patient accommodating means;
said gantry comprising a transporting mechanism set along a longitudinal axis and comprising a proximal portion and a distal portion, said proximal portion being at said second location;
the patient accommodating means comprising a seat at said second location and a handle connected to the seat by a shaped neck;
said open-bore MRD being interconnected to said gantry by at least one maneuverable member whereby said MRD, by means of said gantry, is transportable from said first location to said second location;
the aperture of said MRD's open-bore, by means of said maneuverable member, being directable towards a defined spatial orientation facing the wrist
   of a patient; and said system further comprising a system navigation and orientation mechanism, MRD operating mechanism, seat height adjusters and patient monitors located adjacent to said handle.

Hence, in situ and real time imaging of a static patient who cannot be transferred to MRD's remote location is provided possible.

The aperture of the MRD's open-bore, by means of the maneuverable member, may be directable towards a defined spatial orientation facing the static patient.

The maneuverable member may be selected from a group consisting of an articulated joint, a flexible joint, a rotatable joint, internally compensated self-aligning rotary joint, a ball joint, ball and socket joint, elbow constructed joint, hydraulic joint mechanism, telescopic joint, clampers, gimbals and claspers thereof and any combination thereof.

### BRIEF DESCRIPTION OF THE DRAWINGS

In order to understand the invention and to see how it may be implemented in practice, a few preferred embodiments will now be described, by way of non-limiting example only, with reference to be accompanying drawings, in which:
Fig. 1: shows a system for mobilizing an open-bore MRD (103) having an open bore's aperture (103a) constructed on the ceiling of a patient room;
Fig.2: shows a system for mobilizing an MRD constructed on a wall of a patient room;
Fig. 3: shows a system for mobilizing an MRD constructed on a ceiling of an incubators' room, and where the MRD is set aside;
Fig. 4: shows a system for mobilizing an MRD constructed on a ceiling of an incubators' room, and where the MRD is in use, above one of the incubators;
Fig. 5: shows a system for mobilizing an MRD constructed on a wall of an incubators' room, and where the MRD is in use, above one of the incubators; and
Fig. 6: depicts a flow chart of a method for mobilizing an MRD device;
Fig. 7: illustrates a side view of a maneuverable MRD (here, a wrist imaging device) according to an embodiment of the invention;
Fig. 8: illustrates a rear and side view of a maneuverable MRD according the said embodiment of the invention; and
Fig. 9: illustrates a rear view of a maneuverable MRD according the said embodiment of the invention.

### DETAILED DESCRIPTION OF THE INVENTION

The following description is provided so as to enable any person skilled in the art to make use of the invention and sets forth examples contemplated by the inventor of carrying out this invention. Various modifications, however, will remain apparent to those skilled in the art, since the generic principles of the present invention have been defined specifically. Also, it is to be understood that the phraseology and terminology employed herein is for the purpose of description and should not be regarded as limiting.

A maneuverable MRD system (MMS) for mobilizing an open-bore MRD, characterized by substantially no fringing magnetic fields, in a room towards a static (non-mobile) patient and directing the aperture of the open-bore MRD towards the patient is herein presented. The MMS comprises at least one gantry which is adapted to maneuver the MRD in N degrees of freedom; N is an integer greater than or equal 1.

The term "gantry" refers hereinafter to a crane, or set of cranes, together with a set of modules adapted to move and operate the cranes. The modules may be rails attached to any surface on which cranes are constructed upon. The term also refers hereinafter to a cart, lug, and a wheeled maneuverable construction, such as a forklift truck, forklift, hand pallet truck, such as pallet pump, pump truck, or jigger, walkie low lift truck, rider low lift truck, towing tractor, walkie stacker, rider stacker, reach truck, electric counterbalanced truck, side-loader, telescopic handler, walkie order picking truck, order picker flexi or bendi trucks, guided very narrow aisle truck, guided very narrow aisle order picking truck, truck mounted forklift/sod loader.

The term "maneuverable member" refers hereinafter to a pivoted member, gimbal, joint, articulate structure, rotation or bending constructions etc., which is connected to an object and allows a maneuver the object in one manner or in a multiple degrees of freedom.

The term "location" refers hereinafter to both location and orientation. least one first location to at least one second remote location

The term "patient" refers to a static human patient, such as a neonate, mature patient or portions or organs thereof, such as head, wrist etc. The term further relates to a static animal and organs thereof and to static non-leaving object of any kind, to be analyzed by the moveable MRD.

The term 'static' refers hereinafter to a substantially non-moving, immobilized, static patient and organ thereof.

The term "MRD" refers hereinafter to portable open-bore magnetic resonance imaging device with have no fringing of its magnetic field. The term refers to an MRD which is constructed as a single unit, or a series of units which are not fixed in a certain location. The term also refers to portable and compact MRDs, such as a member of the 'M'-series, commercially available permanent-magnet open-bore MRI device by Aspect Imaging Ltd (US). The term further refers to any analyzing, imaging or scanning medical device, and particularly to nuclear magnetic resonance (NMR) spectroscope, computed tomography (CT), computed axial tomography (CAT), electron spin resonance (ESR) spectroscope, nuclear quadruple resonance (NQR) or to any combination thereof.

The term 'automatic' or 'automatically' refers to any process or action that proceeds without the necessity of direct action of a human being.

The term 'manual' or 'manually 'refers to any process or action necessitating direct operation of a human being, either by physical work, or by operating a computerized system. For example, compact MRD may be maneuvered by pushing it by a medical crew or it can be moved by operating a machine which will move it.

The term "degrees of freedom" refers to the number of independent displacements and/or rotations that specify the orientation of the body or system.

The term "room" refers herein to a predefined room, hallway, space, area, volume, such as a hospital room, intensive care department etc. Moreover, the term room refers herein after to two or more rooms interconnected by a hallway; two or more intercommoned and/or intraconnected spaces etc., such as a hospital's floor (e.g., orthopedic storey), a multi-rooms newborn intensive care unit etc.

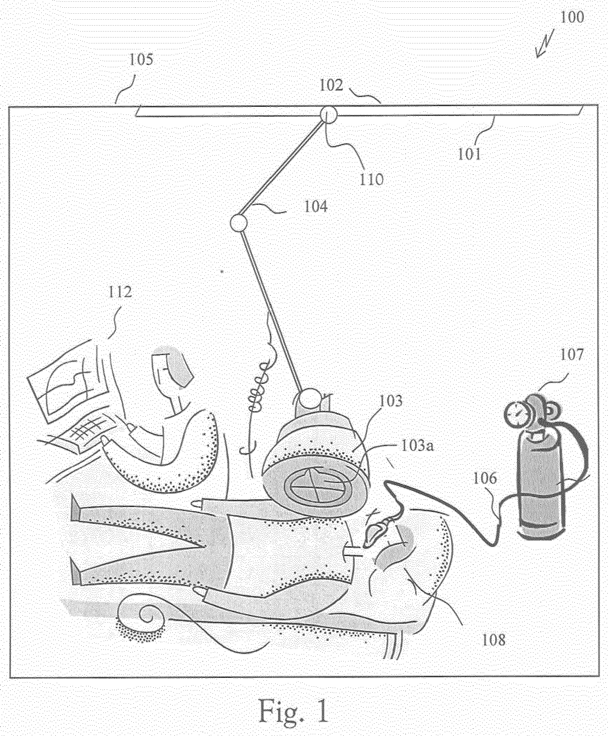

Reference is now made to Fig.1 illustrating in a non-limiting and in non-in-scale manners a system 100 for mobilizing an open bore MRD device 103which comprises an aperture at the end of its bore. System 100 comprises at least one gantry 101 attached to at least one structural surface 102, illustrated as a ceiling; the ceiling is part of a patient's room 105; a compact maneuverable MRD 103device interconnected to at least one gantry 101 such that the gantry 101 is adapted to move this MRD 103in N degrees of freedom; Nis an integer greater than or equals 1.

Still referring Fig. 1, an MRD maneuvering system (MMS) 100 is useful for mobilizing MRD 103 in a room 105. This MMS comprises, in a room (105) comprising a structural surface (102), a gantry (101) temporarily or permanently interconnected to the surface (or to the room's infrastructure) by a transporting mechanism (e.g., wheels 110); and an MRD (103) characterized by a non-fringing magnetic field, having open-bore's aperture (103a)in which patient or organ thereof is imagined, temporarily or permanently interconnected to the gantry by at least one joint (104). At at least one second location adjacent to a static (non-moving) patient, the maneuverable MRD, by means of the gantry or the joint (e.g., 110and/or 104), is adapted to be (i) positioned and (ii) spatially oriented in two or more different orientations; and wherein the gantry, by means of the transporting mechanism, is adapted to reciprocate the MRD from the at least one first location to at least one second remote location.

Compact MRD 103is interconnected to the at least one gantry 101by a module 104constructed of N joints (e.g., 1< N>4) as schematically illustrated in Fig. 1.

Further illustrated in Fig. 1, is a static immobilized patient 108, e.g., back-injured patient, neonate etc., who is statically positioned within room 105or laying within his/her life supporting environment, incubator etc., and connected via tubes 106to medical aid 107 (e.g. oxygen, blood infusion). Gantry 101 delivers the maneuverable MRD 103 directly towards this non-moving patient 108 and provides the imaging without disconnecting the patient from his/her medical aid 107; thereby provides the patient with an in situ, real time (in vivo) imaging without mobilizing the patient to a remote location where MRDs usually placed and possibly deteriorating his health condition.

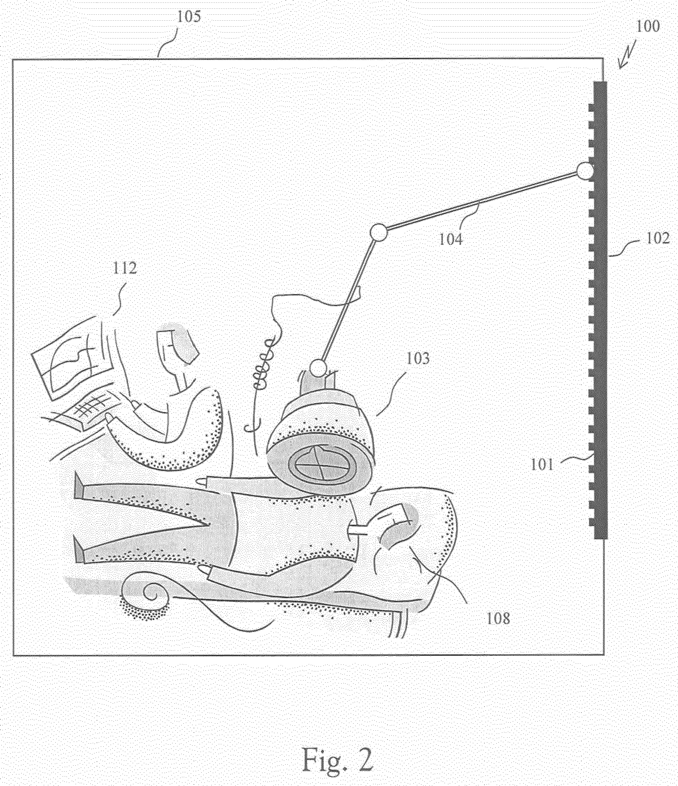

Reference is now made to Fig. 2, illustrating MMS 100 which is constructed in a patient's 108 room 105. MMS 100 comprises a gantry 101 (here with 3 joints module 104), attached to the room's 105 structural surface 102, here, a wall. The joints' module 104 is connected to a compact maneuverable MRD 103. The gantry 101 is adapted to move this MRD 103 in N degrees of freedom. Also illustrated in Fig. 2, is a computer station 112 in communication with MRD 103. The computer station instructs the movable MRD for imaging of the static patient 108 without moving the patient 108.

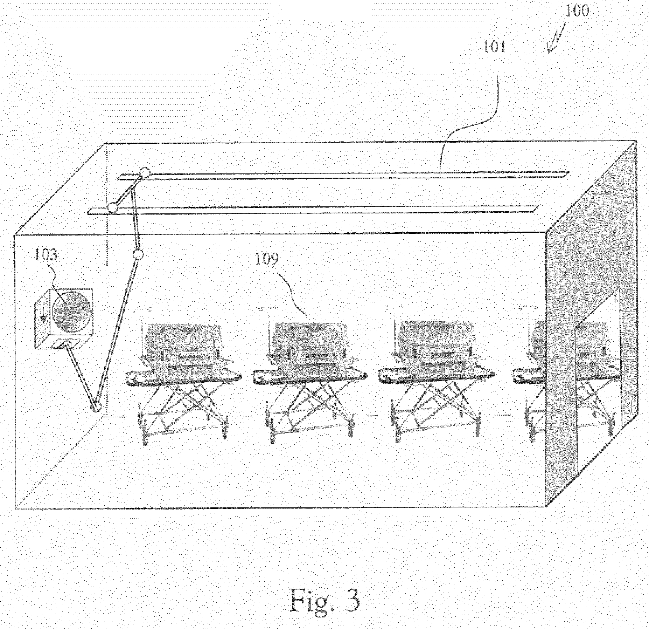

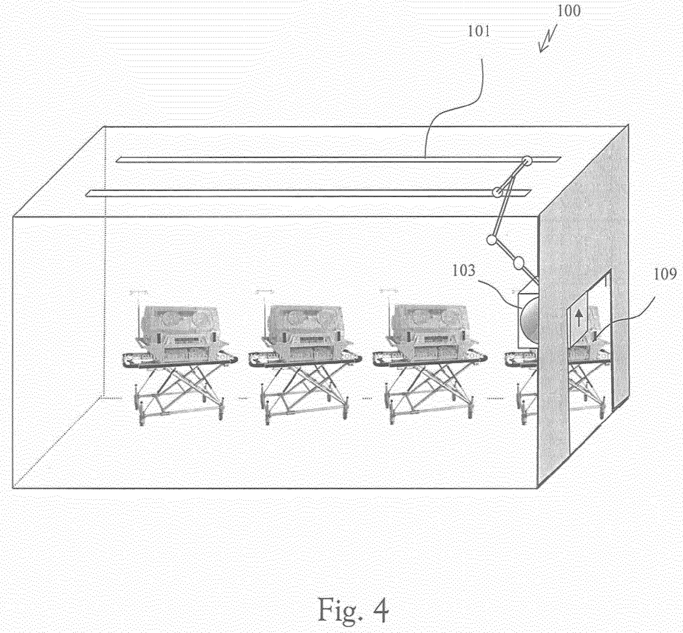

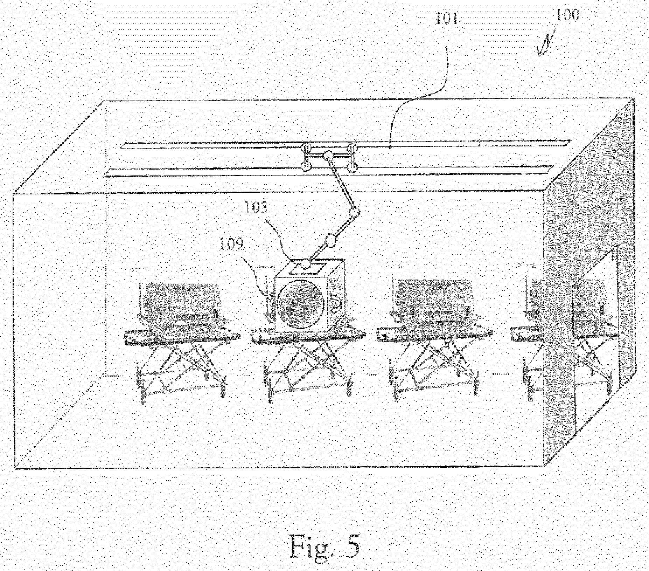

Reference is now made to Fig. 3 illustrating MMS (100) constructed on a ceiling of neonates incubators' room; the neonate-type MRD (103) is set aside between uses. It is noted that the MRD is located here in a first location (up left) and spatially oriented in a first spatial orientation. Fig. 4 demonstrates MMS (100) in use, above an incubator (109). It is further noted that the MRD is located here in a second remote location (low right) and spatially oriented in a second spatial orientation. Reference is now made to Fig. 5 illustrating in a non-limiting manner another embodiment of the current invention where MMS (100) is constructed on a wall of an incubators' room; the MRD (103) is demonstrated in use, above a neonate's incubator (109). It is further noted that the MRD is located here in a third remote location (middle) and the MRD's open bore's aperture is spatially oriented in a third spatial (i.e., tilted-) orientation towards the neonate.

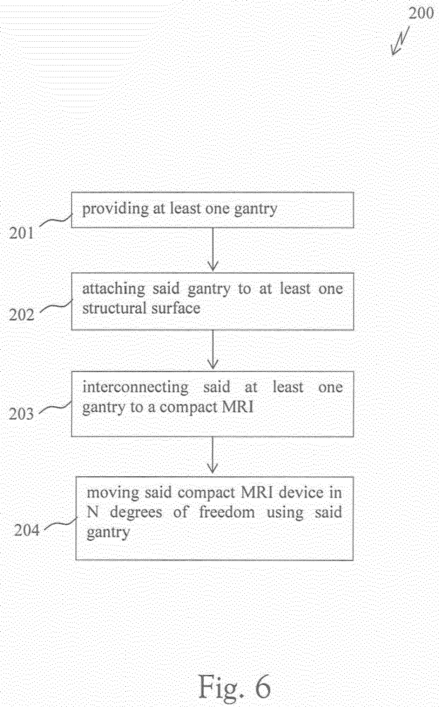

Reference is now made to Fig. 6, illustrating a flow chart of one method 200 for mobilizing an MRD, the method comprises step 201 of providing at least one gantry; step 202 of attaching the gantry to at least one structural surface; step 203 of interconnecting at least one gantry to a compact MRD. This method additionally comprises a step 204 of moving the compact MRD in N degrees of freedom using the gantry: e.g., moving the MRD (along plain X,Y), elevating the device along the Z axis, and rotating it along said Z axis, to the static, non-mobile patient, and then directing the MRD's open-bore's aperture towards the still immobilized patient, by maneuvering MRD along XY, YZ, and XZ plains, and possibly further rotating or tilting the same (at any θ) along or around any required 2D or 3D axis.

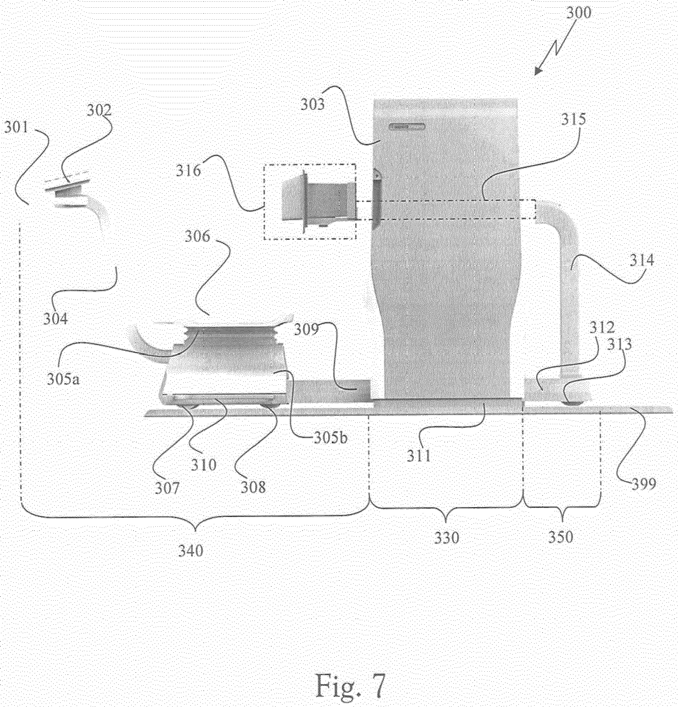

Reference is now made to Fig. 7, illustrating a side view of a maneuverable MRD system (MMS, 300) comprising a maneuverable MRD (303) here, for example, an open-bore permanent-magnet wrist-type MRI device, commercially available by Aspect Imaging Ltd, US, according to an embodiment of the invention. The MRD is set along a main longitudinal axis (see middle portion 330); and an MRD mobilizing mechanism (herein provided in a non-limiting manner a manual jigger-type gantry) comprising a proximal portion (340), and a distal portion (350), neighboring central MRD's portion 340.MMS (300) comprises *inter alia*, in its proximal portion 340, patient's accommodating means, such as MMS's handle (301) which is a U-shape member, s leading rod, guiding reed, steering wheel-like member etc. Handle (301) is connected to patient's seat (360) by means of a shaped-neck (e.g., S-shape member 306). MMS' navigation and operation mechanism, MRD operating mechanism, patient's-seat height adjusters, as well as patient's monitors (302) are locatable adjacent to handle 301. Seat's (306) elevation and orientation is definable by means of a seat maneuvering mechanism, which comprises mechanism 305 alocated within base (305b). Patient's leg acceptors (310) are located aside base 305b. A first set of wheels (307, 308) are affixed, e.g., under the base, on the floor (399). The floor 399 here is a structural surface, and the MRD-connecting gantry is maneuvering the MRD towards the statistic patient's organ over the floor by means of wheels, rail and by means of elevation mechanism 305a etc.

Still along said main longitudinal axis, a set off one or more rails is provided to bear MRD's weight. This MMS's elongated skeleton comprises a proximal portion (309), central portion (311), located under the MRD, and a distal portion (312). A second set of wheels (313)is provided at the distal side of the MMS (350).Construction member 314is erected and curved to support, either in or adjacent to MRD's open bore (315)wrist accepting assembly (316). It is in the scope of the invention wherein the hereto disclosed floor-gantry comprises one or more maneuverable members, e.g., a joint provided between base (305b) and rail 309. It is also in the scope of the invention wherein the hereto disclosed floor-gantry comprises at least one articulating mechanism, at least one rotation, elongation or bending mechanisms, e.g., a joint provided between base (305b) and rail 309; elevation mechanism of shaft 305a etc.

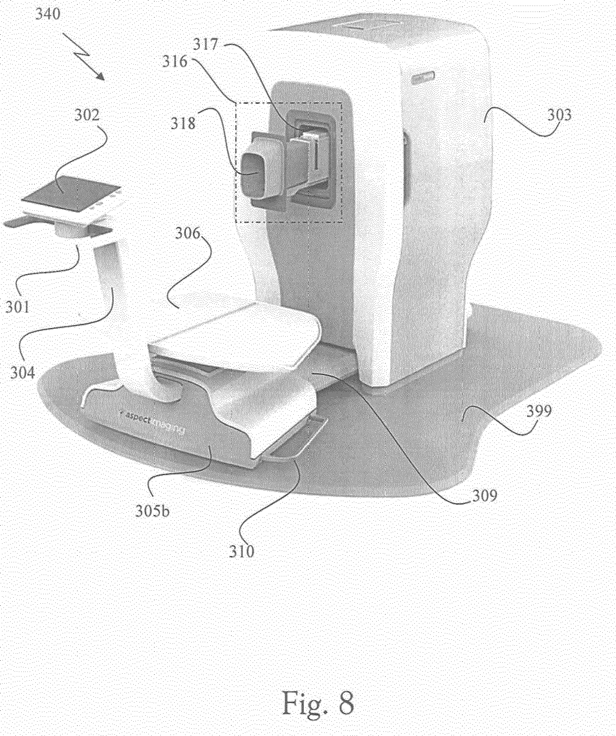

Reference is now made to Fig. 8, which illustrates a rear and side view of the proximal side (340) of MMS (300) defined above. Here again, along the main longitudinal axis there is located handle (301) and monitors/operators (302), neck (304) interconnected to a seat's base (305b) upon which the patient rest (see seat 306). A pair of legs rest (310) is there provided. MMS's elongated lower skeleton (309, 311and 312) is carrying an MRD (303), here an open-bore's aperture (318) of a permanent-magnet MRI device comprising a wrist neck (317) provided as an integral member of a wrist accepting assembly (316).

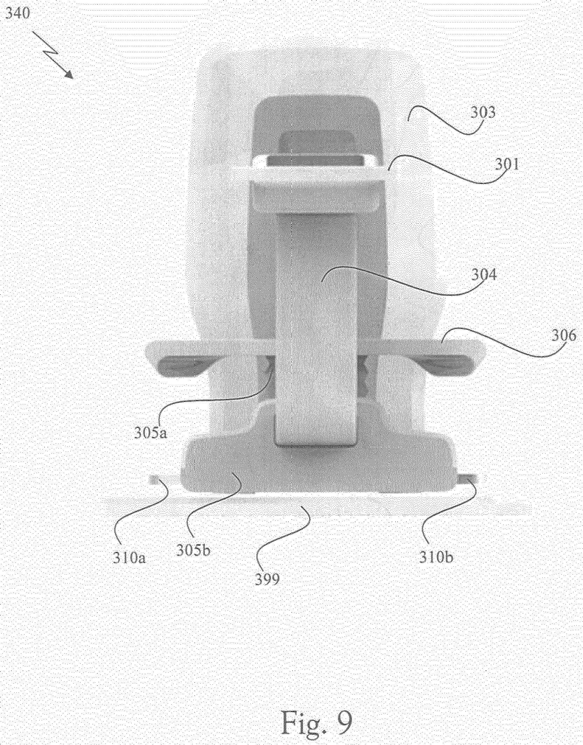

Reference is now made to Fig. 9, which illustrates a rear-view of the proximal side (340) of MMS (300) which is defined above. MRD (303) is maneuverable transported by the MMS, comprising, inter alia, in its proximal portion a handle (301), neck (304), connected to a base (305b) which support seat (306) by means of reciprocating (along Z axis) and rotating (around Z axis) mechanism (305a). A pair of legs rests (310a and 310b) is further provided.

It is in the scope of the invention wherein to three different MMSs are provided useful: in a first MMS, the MRD is moving in respect to a non-moving (immobilized static) patient or non-moving patient's organ to be imagined. In a second MMS, the MRD is moving in respect to a moving (non-immobilized) patient or moving (non-immobilized) patient's organ to be imagined. Hence for example, second MMS is applicable in a system where a movable MMS is utilized to scan an organ of a patient (e.g., his/her wrist): the patient is static yet his/her organ is movable. In a third MMS, the MRD's envelope is static yet the organ's accepting assembly of the MRD is movable. Here again, the patient can be either static or mobile.

In an embodiment of the invention, a self-fastening cage (SFC) of an MRD is utilized in an MMS as defined above. This SFC-MRD is characterized by an outside shell, which comprises at least three flexi-jointed superimposed walls disposed in a predetermined clockwise or counterclockwise arrangement. The MRD comprises at least six side-magnets arranged in two equal groups being in a face-to-face orientation in a magnetic connection with the outside shell, increasing the overall strength of the magnetic field provided in the cage; at least two pole-magnet pieces, arranged in a face-to-face orientation in between the side-magnets; and, at least two main-magnets, located on the pole-pieces, arranged in a face-to-face orientation, generating the static magnetic field therein the cage. As an example, at least a portion of the side-magnets are superconductors or ferromagnets. A commercially available selected from the M-series product of Aspect Imaging Ltd (US) is an example of such an SFC-MRD.

In some embodiments of the current invention, the degrees of freedom as defined above, which the gantry enables may be selected from a group consisting for example of: horizontal (X axis and Y axis), vertical (Z axis), longitudinal, altitudinal, angular, and a combination thereof

In some embodiments of the current invention, movement of the compact MRD as defined above, is provided in a manual manner, automatic mechanism, computerized mechanism, and any combination thereof

In some embodiments of the current invention, the structural surface as defined above may be selected from a group consisting of: floor, ceiling, wall, any constructed surface, and any combination thereof

In some embodiments of the current invention, compact MRD as defined above, is interconnected to at least one gantry by a module constructed of N joints. In other embodiments, a maneuverable member may be constructed as part of the gantry. In yet another embodiment, the gantry may be modular, enabling the removal of certain parts upon request.

In some embodiments of the current invention, the gantry as defined above, may be located in a room selected from a group consisting for example of: hospital room, premature special care unit, intensive care unit, neonatal unit, any space in which lies a disable patient, immobilized objet, and a combination thereof.

In some embodiments of the current invention, compact MRD as defined above, is in communication with a working station adapted to operate the compact MRD.

### EXAMPLE 1

In one example outside the scope of the claimed invention, a premature is in an incubator in a newborn intensive care unit. The premature neonate needs an MRD scanning, however, moving them to an MRD located in a different room, and usually to a different department in different floor is very dangerous.

Thus, in the newborn intensive care unit, an MMS for maneuvering a neonate's-type MRD is provided. The MMS comprises a gantry which comprises a transporting mechanism and, a neonate's-type MRD, interconnected to the gantry by at least one maneuverable member. The MRD, by means of the gantry, is transportable from a first location to or adjacent a static neonate located at a second remote location, where neonate's incubator is placed. The aperture of the neonate's-type MRD's open-bore, by means of the maneuverable member, is directable towards a defined spatially orientation facing the neonate or incubator thereof. Thus, the premature neonate is not mobilized nor transferred from his safe environment to a remote dangerous location where MRD usually maintained and operated.

The imaging of this premature neonate in his incubator is provided useful by a method of maneuvering a portable open-bore neonate's-type magnetic resonance device with no fringing of its magnetic field (neonate's-type MRD) from at least one first location towards at least one static neonate placed in an incubator at least one second remote location by means of a neonate's-type MRD maneuvering system (MMS). The method comprises inter alia steps as follows; providing an MMS with in a room; interconnecting the MMS to a gantry having inter alia a transporting mechanism; interconnecting an open-bore neonate's-type MRD to the gantry by at least one maneuverable member; transporting the neonate's-type MRD, by means of the gantry, from the first location to the second remote location adjacent to a neonate or incubator thereof; and directing the aperture of the neonate's-type MRD's open-bore, by means of the maneuverable member, towards a defined spatially orientation facing the neonate or an incubator thereof.

It is acknowledged in this respect that a method of mobilizing a manuverable open-bore MRD from a first location to a second remote location to a static trauma patient or organs thereof is further useful. This method is characterized by the following steps: providing in the intensive care unit at least one first location and at least one second remote location, namely the patient bad and medical facilities, life supporting systems, and tubing thereof; constructing a structural surface in at least one portion of the intensive care unit; temporarily or permanently interconnecting the gantry to the surface by means of a transporting mechanism; temporarily or permanently interconnecting the gantry to an MRD by means of at least one manuverable member; transporting the MRD, by means of the transporting mechanism, from the at least one first location to the at least one second remote location; and at the at least one second location, spatially orienting the open-bore's aperture of the MRD, by means of the manuverable member, towards the trauma patients or organs thereof; thereby imaging this immobilized trauma patient or organ thereof in vivo and in situ by means of this maneuverable MRD.

### EXAMPLE 2

In another example outside the scope of the claimed invention, after a trauma caused by a serious car accident, a patient in coma is medically treated in an intensive care unit. MRI is required, nevertheless, he is respirated, and is connected to a plurality of life supporting systems and pings thereof; therefore he cannot be moved from his bed.

Hence, by a means of a gantry of the present invention, which is provided useful for maneuvering a portable open-bore magnetic resonance device with no fringing of its magnetic field (MRD) from at least one first location towards at least one static patient placed at at least one second remote location. Here, the remote location is the trauma quarters in the intensive care unit. The gantry comprises a transporting mechanism; and, an open-bore MRD, interconnected to the gantry by at least one maneuverable member. The MRD, by means of the gantry, is transportable from the first location to the second remote location adjacent the static patient. The aperture of the MRD's open-bore, by means of the maneuverable member, is directable towards a defined spatially orientation facing the static patient.

The imaging of this trauma patient is provided useful by a method of maneuvering a portable open-bore magnetic resonance device with no fringing of its magnetic field (MRD) from at least one first location towards at least one static patient placed at at least one second remote location by means of a gantry. The method comprises, inter alia, steps as follows: providing the gantry with a transporting mechanism; interconnecting an open-bore MRD to the gantry by at least one maneuverable member; transporting the MRD, by means of the gantry, from the first location to the second remote location adjacent the static patient; and directing the aperture of the MRD's open-bore, by means of the maneuverable member, towards a defined spatially orientation facing the static trauma patient.

### EXAMPLE 3

In an example of the current invention, in an orthopedic clinic, (i) a wrist-type MRD is maneuverable in respect to a sitting (non-moving) patient with a broken hand to be analyzed; (ii) a wrist-type MRD is maneuverable in respect to a sitting (yet moving) patient with a broken hand to be analyzed. In the first case, the MRD of the open-bore portion of the same is maneuverable in respect to the wrist to be imagined.

For this purpose, an MMS for maneuvering a wrist-type MRD is provided. The MMS comprises inter alia: a gantry which further comprises a transporting mechanism; and, a wrist-type MRD, interconnected to the gantry by at least one maneuverable member; wherein the MRD, by means of the gantry, is transportable from a first location to a second remote location; and further wherein the aperture of the MRD's open-bore, by means of the maneuverable member, is directable towards a defined spatially orientation facing the wrist of a patient.

The imaging of this wrist is provided useful by a method of maneuvering a portable open-bore wrist-type magnetic resonance device with no fringing of its magnetic field (wrist-type MRD) from at least one first location towards at least one static patient placed at at least one second remote location by means of a wrist-type MRD maneuvering system (MMS). This method comprising steps of providing the MMS with in a room; interconnecting the MMS to a gantry having inter alia a transporting mechanism; interconnecting an open-bore wrist-type MRD to the gantry by at least one maneuverable member; transporting the wrist-type MRD, by means of the gantry, from the first location to the second remote location adjacent to the static patient and directing the aperture of the wrist-type MRD's open-bore, by means of the maneuverable member, towards a defined spatially orientation facing the patient's wrist.

## Claims

1. A maneuverable system (300) comprising:
a maneuverable open-bore wrist-type magnetic resonance device (303) with no fringing of its magnetic field in which the open bore comprises an aperture (318),
a gantry for maneuvering said magnetic resonance device (303) from at least one first location towards at least one second location with patient accommodating means;
said gantry comprising a transporting mechanism set along a longitudinal axis and comprising a proximal portion (340) and a distal portion (350), said proximal portion being at said second location;
the patient accommodating means comprising a seat (360) at said second location and a handle connected to the seat by a shaped neck;
said magnetic resonance device (303) being interconnected to said gantry (101) by at least one maneuverable member;
the aperture of said magnetic resonance device's open-bore, by means of said maneuverable member, being directable towards a defined spatial orientation facing the wrist of a patient; and said system further comprising a system navigation and orientation mechanism, a magnetic resonance device operating mechanism, seat height adjusters and patient monitors (302) located adjacent to said handle.

2. The system according to claim 1, comprising a wrist neck (317) provided as an integral member of a wrist accepting assembly (316) at the open bore aperture.

3. The system according to any preceding claim, wherein said maneuverable member is selected from a group consisting of an articulated joint, a flexible joint, a rotatable joint, internally compensated self-aligning rotary joint, a ball joint, ball and socket joint, elbow constructed joint, hydraulic joint mechanism, telescopic joint, clampers, gimbals and claspers thereof and any combination thereof.

4. The system according to any preceding claim comprising a seat maneuvering mechanism for defining seat height and orientation comprises a mechanism (305a) located within a base (305b) and set of wheels (307, 308) affixed under the base.

5. The system according to any preceding claim wherein along the longitudinal axis the system further comprises a set of rails to bear the weight of said magnetic resonance device.

## Patentansprüche

1. Ein verfahrbares System (300), das Folgendes aufweist:
eine verfahrbare offene handgelenkartige Magnetresonanzvorrichtung (303) ohne Streuung ihres Magnetfeldes, in der das offene Profil eine Öffnung (318) aufweist,
ein Gerüst zum Verfahren der besagten Magnetresonanzvorrichtung (303) von mindestens einem ersten Standort zu mindestens einem zweiten Standort mit Patienten-Aufnahmevorrichtung;
das besagte Gerüst weist dabei einen Transportmechanismus auf, der entlang einer Längsachse justiert ist und einen proximalen Teil (340) und einen distalen Teil (350) aufweist, der besagte proximale Teil befindet sich am zweiten Standort;
die Patienten-Aufnahmevorrichtung weist einen Sitz (360) am zweiten Standort und einen Griff auf, der mit dem Sitz über ein geformten Stutzen verbunden ist;
diese Magnetresonanzvorrichtung (303) ist mit dem besagten Gerüst (101) über mindestens ein verfahrbares Teil verbunden;
die Öffnung des offenen Profils der Magnetresonanzvorrichtung ist mittels des verfahrbaren Teils in eine definierte räumliche Richtung ausrichtbar, die auf das Handgelenk eines Patienten zeigt; und das besagte System weist darüberhinaus einen Systemnavigations- und Systemausrichtungsmechanismus, einen Betätigungsmechanismus der Magnetresonanzvorrichtung, Sitzhöhen-Einstellvorrichtungen und Patientenmonitore (302) auf, die neben dem Griff angebracht sind.

2. Das System nach Anspruch 1, das einen Handgelenk-Stutzen (317) aufweist, der als eingebauter Teil einer Handgelenk-Aufnahmevorrichtung (316) am offenen Profil der Öffnung angebracht ist.

3. Das System nach einem der vorhergehenden Ansprüche, wobei der verfahrbare Teil ausgewählt ist aus der Gruppe bestehend aus einem Knickgelenk, einem elastischen Gelenk, einem Drehgelenk, einem intern kompensierten selbstausrichtenden Drehgelenk, einem Kugelgelenk, Ellbogengelenk, Hydraulikgelenkmechanismus, Teleskopgelenk, deren Schellen, Aufhängeringe und Hakenverschlüsse und jede Kombination daraus.

4. Das System nach einem der vorhergehenden Ansprüche, das einen Sitzverfahrmechanismus für die Einstellung der Sitzhöhe und -ausrichtung aufweist und einen Mechanismus (305a) im Sockel (305b) und einen Satz Räder (307, 308), die unter dem Sockel angebracht sind.

5. Das System nach einem der vorhergehenden Ansprüche, wobei entlang der Hauptlängsachse das System darüberhinaus einen Satz Schienen aufweist, die das Gewicht der Magnetresonanzvorrichtung tragen.

## Revendications

1. Un système manoeuvrable (300) consistant en :
un dispositif à résonance magnétique manoeuvrable à alésage ouvert de type poignet (303) ne comportant aucune frange de son champ magnétique dans lequel l'alésage ouvert comprend une embouchure (318),
un portique destiné à manoeuvrer ledit dispositif à résonance magnétique (303) à partir d'au moins un premier emplacement vers au moins un second emplacement comportant le moyen d'accueil du patient pouvant ;
ledit portique comprenant un mécanisme de transport établi le long d'un axe longitudinal et comprenant une partie proximale (340) et une partie distale (350), ladite partie proximale étant au niveau dudit second emplacement;
le moyen d'accueil du patient consistant en un siège (360) au niveau dudit second emplacement et une poignée liée au siège par un collier façonné ;
ledit dispositif à résonance magnétique (303) étant interconnecté audit portique (101) par au moins un élément manoeuvrable ;
l'embouchure dudit alésage ouvert du dispositif à résonance magnétique, au moyen dudit élément manoeuvrable, étant dirigée vers une orientation spatiale définie en regard du poignet d'un patient ; et ledit système comprenant en outre un système de navigation et un mécanisme d'orientation, un mécanisme d'exploitation d'un dispositif à résonance magnétique, des rehausseurs de siège et des moniteurs pour le patient (302) situés adjacents à ladite poignée.

2. Le système selon la revendication 1, comprenant un collier de poignet (317) fourni comme une partie intégrante d'un ensemble acceptant le poignet (316) à l'embouchure de l'alésage ouvert.

3. Le système selon une quelconque revendication précédente, dans lequel ledit élément manoeuvrable est sélectionné parmi un groupe composé d'une articulation articulée, une articulation flexible, une articulation rotative, une articulation tournante autoalignée compensée en interne, une articulation à rotule, une articulation sphéroïde, une articulation de coude construite, un mécanisme d'articulation hydraulique, une articulation télescopique , des préhenseurs, des cardans et des organes de serrage de ceux-ci et toute combinaison de ceux-ci.

4. Le système selon une quelconque revendication précédente consistant en un mécanisme de manoeuvre du siège destiné à définir la hauteur et l'orientation du siège consiste en un mécanisme (305a) situé à l'intérieur d'une base (305b) et de l'ensemble de roues (307, 308) fixé sous la base.

5. Le système selon une quelconque revendication précédente, dans lequel le long de l'axe longitudinal principal, le système consiste en outre en un ensemble de rails pour supporter le poids dudit dispositif à résonance magnétique.
